# FASCICULE DE BREVET EUROPEEN

(11) **EP 1 092 402 B1**
(45) Date de publication et mention de la délivrance du brevet: **09.03.2005**
(21) Numéro de dépôt: 00402801.5
(22) Date de dépôt: 11.10.2000
(51) Int. Cl.: A61F 2/16

(54) **Implant intraoculaire prismatique pour chambre postérieure de l'oeil**
Prismatische Hinterkammerlinse
Prismatic intraocular lens for the posterior chamber of the eye

(30) Priorité: 13.10.1999 FR 9912767
(43) Date de publication de la demande: 18.04.2001
(73) Titulaire: Ioltechnologie-Production, 17000 La Rochelle (FR)
(72) Inventeur: Colin, Joseph, 29800 Bohars (FR)
(74) Mandataire: Santarelli

(56) Documents cités:
- EP-A- 0 749 732
- WO-A-91/13597
- WO-A-98/05272
- WO-A-98/44875
- US-A- 4 581 031
- US-A- 5 769 890
- US-A- 5 968 094

## Description

La présente invention concerne un implant intraoculaire pour chambre postérieure de l'oeil.

Elle s'inscrit de manière générale dans le domaine de l'implantation de lentilles intraoculaires dans la chambre postérieure de l'oeil dans le cas de pathologies maculaires.

Lorsque des patients sont atteints d'une cataracte, la déficience visuelle est telle qu'ils sont proches de la cécité.

Généralement, on pratique l'exérèse du cristallin et on implante dans la chambre postérieure de l'oeil un implant intraoculaire dont l'élément optique permet à la fois de dévier le faisceau optique sur une zone maculaire saine et d'apporter une correction dioptrique.

Un tel implant intraoculaire est décrit notamment dans le brevet américain N° 4 581 031, ainsi que dans les demandes de brevet international N° WO 98/05272 et WO 98/44875. L'implant intraoculaire comporte une portion prismatique pour diriger les rayons lumineux sur une zone saine de la macula et une portion convexe adaptée à focaliser les rayons lumineux pour remplacer la fonction réfractive du cristallin naturel.

La portion prismatique faisant partie du même implant intraoculaire que la portion convexe, elle est implantée en même temps que la portion convexe au moment de l'extraction du cristallin. En outre, un tel implant intraoculaire prismatique, de par sa conception, est un implant de taille importante du fait notamment de l'association d'un prisme avec une portion de lentille convexe.

Il arrive en outre parfois que cette correction prismatique soit mal supportée par le patient et que l'on soit obligé d'explanter l'implant intraoculaire.

Le retrait de cet implant prismatique est également rendu nécessaire lorsque la zone maculaire malade se modifie et que l'on doit modifier la correction prismatique pour dévier le faisceau optique sur une nouvelle zone maculaire saine.

Le but de la présente invention est de proposer un implant intraoculaire prismatique pour chambre postérieure de l'oeil qui permette d'apporter de manière simple une correction prismatique.

A cet effet, l'implant intraoculaire pour chambre postérieure de l'oeil visé par l'invention comprend un élément optique et des éléments haptiques s'étendant à la périphérie de l'élément optique.

Conformément à l'invention, cet élément optique, ne comprend qu'une portion prismatique, et est adapté uniquement à dévier un faisceau optique sur une zone maculaire saine.

On peut ainsi apporter une correction prismatique pour la déviation du trajet optique dans l'oeil de manière indépendante à toute correction dioptrique.

L'élément optique de l'implant intraoculaire conforme à l'invention ne comprenant qu'une portion prismatique est de taille moins importante que les implants prismatiques utilisés à ce jour.

Cet implant intraoculaire permet en outre d'équiper des patients déjà opérés de la cataracte en ajoutant uniquement un implant à correction prismatique.

Selon une caractéristique préférée de l'invention, cet implant intraoculaire prismatique constitue un ensemble d'implants intraoculaires avec un implant principal qui est adapté à remplacer le cristallin et préimplanté dans la chambre postérieure de l'oeil.

Ainsi, si la correction prismatique doit être modifiée, seul l'implant intraoculaire à portion prismatique conforme à l'invention peut être explanté de l'oeil sans intervenir sur l'implant principal préimplanté. Cette explantation est aussi considérablement facilitée.

La correction dioptrique n'est par conséquent pas modifiée.

Suivant un autre aspect de l'invention, les éléments haptiques de l'implant prismatique conforme à l'invention sont adaptés à coopérer avec des éléments haptiques de l'implant principal préimplanté.

Ce montage est particulièrement bien adapté lorsque l'on doit changer l'implant prismatique pour modifier la déviation du faisceau lumineux, puisqu'il n'est pas nécessaire d'intervenir sur des tissus oculaires contrairement aux implants dont les haptiques viennent en contact avec ces tissus.

De manière avantageuse, qui permet un montage relativement simple de l'implant conforme à ce mode préféré de réalisation, les éléments haptiques sont des anses adaptées à s'entrecroiser avec des éléments haptiques en forme d'anses de l'implant principal préimplanté.

Suivant une autre caractéristique avantageuse de l'invention, l'élément optique est réalisé en un matériau d'indice de réfraction élevé.

Grâce à cet indice de réfraction élevé, l'inclinaison du prisme nécessaire à une bonne déviation, peut être faible, ce qui limite son encombrement dans la chambre postérieure de l'oeil.

D'autres particularités et avantages de l'invention apparaîtront encore dans la description ci-après.

Aux dessins annexés, donnés à titre d'exemples non limitatifs :
- la figure 1 est une vue de face d'un implant intraoculaire conforme à un premier mode de réalisation de l'invention ;
- la figure 2 est une vue de côté de l'implant de la figure 1 ;
- la figure 3 est une vue en coupe d'un oeil illustrant l'implantation de l'implant de la figure 1 ;
- la figure 4 est une vue de face d'un implant intraoculaire conforme à un second mode de réalisation de l'invention ;
- la figure 5 est une vue de face d'un implant formant lentille monofocale adaptée à supporter l'implant de la figure 4 ;
- la figure 6 est une vue de face des implants illustrés aux figures 4 et 5 fixés l'un à l'autre ; et
- la figure 7 est une vue de côté des implants dé la figure 6 mis en place dans l'oeil;
- la figure 8 est une vue de face d'un support adapté à porter deux implants associés;
- la figure 9 est une vue de face d'un implant intraoculaire associé à un implant formant lentille monofocale selon un troisième mode de réalisation de l'invention;
- la figure 10 est une vue en coupe selon la ligne A-A à la figure 9;
- la figure 11 est une vue en coupe selon la ligne B-B à la figure 9; et
- la figure 12 est une vue en coupe d'un oeil illustrant l'implantation de l'implant de la figure 9.

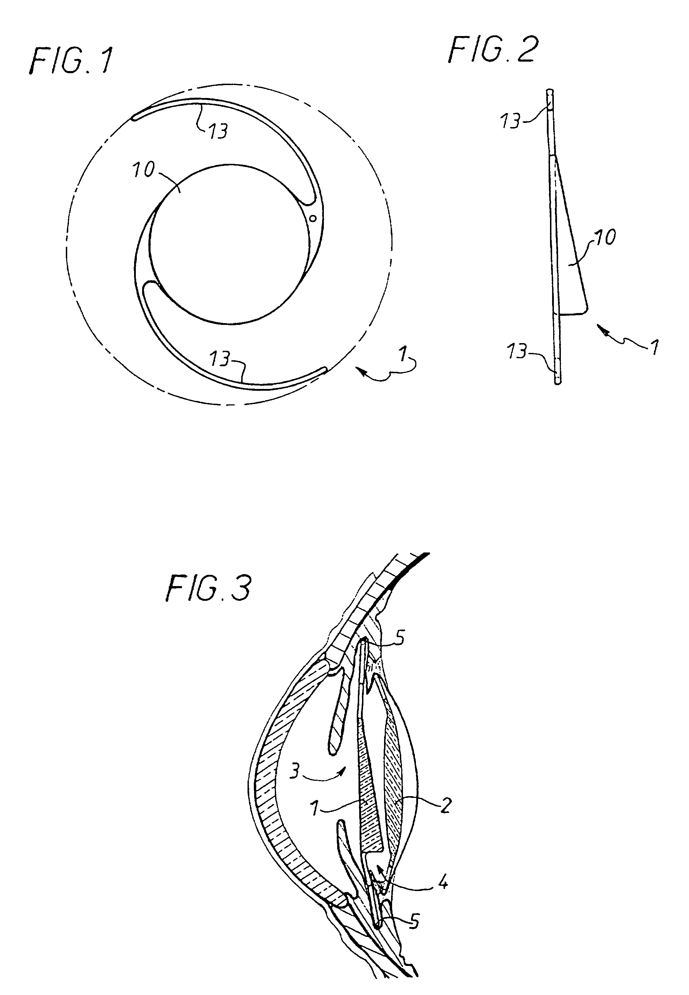

On va décrire tout d'abord la structure d'un implant intraoculaire conforme à un premier mode de réalisation de l'invention, illustré aux figures 1 et 2.

Cet implant intraoculaire 1 comprend un élément optique 10 et des éléments haptiques 13 qui s'étendent à la périphérie de l'élément optique 10.

L'élément optique 10, comme bien illustré à la figure 2, comprend seulement une portion prismatique adaptée à dévier le faisceau optique sur une zone maculaire saine.

Cet élément optique prismatique 10 permet ainsi de modifier le point d'impact sur la macula du faisceau de lumière arrivant dans l'oeil.

Il est important de choisir pour la fabrication de cet implant un matériau ayant un indice de réfraction élevé. En effet, plus son indice est élevé, moins l'inclinaison du prisme est nécessaire, ce qui limite son encombrement.

A titre d'exemple, on peut utiliser des matériaux rigides qui ont généralement un indice de réfraction plus élevé.

Bien entendu, le matériau utilisé doit être stable dans le temps, non cataractogène, et non irritant.

Afin de faciliter sa mise en place et la tolérance dans l'oeil, on recherche également un matériau d'une souplesse et d'une hydrophilie satisfaisantes.

On peut utiliser à titre d'exemple un acrylique hydrophile tel qu'un copolymère hydroxyéthyl méthylméthacrylate.

La matière utilisée pourrait également être de caractère hydrophobe.

Les éléments haptiques 13 de l'implant intraoculaire 1 sont constitués dans cet exemple d'anses 13 qui s'étendent dans le même sens (par exemple dans le sens inverse des aiguilles d'une montre dans la vue de face à la figure 1) à partir de l'élément otique 10.

Ces anses 13 sont diamétralement opposées et ont une épaisseur de l'ordre de 0,2 mm.

L'élément optique 10 est sensiblement circulaire dans son plan et a un diamètre égal par exemple à 6 mm.

Comme illustré à la figure 3, cet implant intraoculaire conforme à l'invention 1 est placé en position antérieure dans la chambre postérieure 3 de l'oeil alors qu'un implant principal 2, implanté en premier, est en position postérieure.

Dans cet exemple, l'implant principal 2 est un implant formant lentille intraoculaire monofocale adaptée à remplacer le cristallin.

Il est implanté dans le sac capsulaire 4 et vient en appui entre la capsule antérieure et la capsule postérieure du sac capsulaire 4. Cet implant principal 2 possède ainsi un élément optique formé d'une lentille biconvexe adaptée à remplacer de manière efficace le cristallin.

Dans ce mode de réalisation de l'invention, l'implant intraoculaire 1 conforme à l'invention est alors implanté dans le sulcus ciliaire 5.

Pour obtenir un appui correct de cet implant intraoculaire 1 dans le sulcus ciliaire 5, il présente une dimension hors tout égale à environ 12 mm, qui corrspond ici à la distance séparant les anses 13 de l'implant 1.

De préférence, la portion inclinée de la lentille prismatique 10 de l'implant 1 est dirigée postérieurement dans l'oeil, afin d'éviter tout contact avec l'iris.

Ces deux implants 1, 2, doivent également être réalisés en un matériau suffisamment stable.

En particulier, des anses rigides 13 offrent une meilleure stabilité à l'implant intraoculaire 1 pour son implantation dans le sulcus ciliaire.

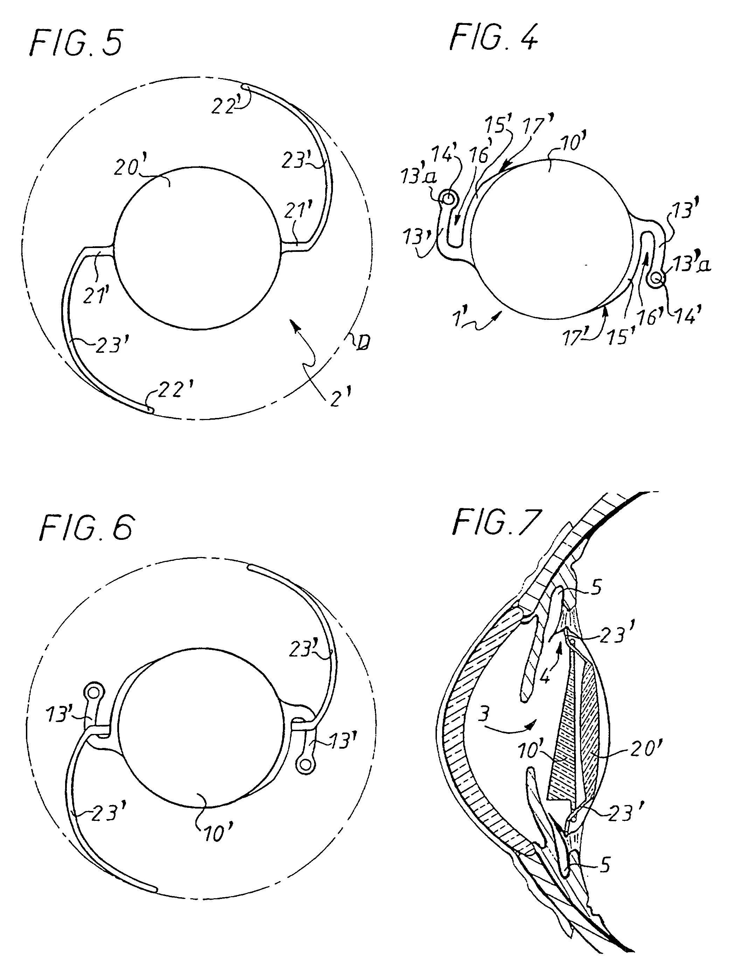

On va décrire à présent un second mode de réalisation de l'invention en référence aux figures 4 à 7.

Selon ce mode de réalisation de l'invention, l'implant intraoculaire 1', illustré à la figure 4, forme une lentille intraoculaire prismatique adaptée à être montée sur un implant principal 2', illustré à la figure 5.

L'implant intraoculaire 1' comprend, comme précédemment, un élément optique 10' constitué uniquement d'une portion prismatique comme illustré en coupe à la figure 7.

Cet implant intraoculaire 1' a uniquement pour rôle de dévier le faisceau lumineux dans l'oeil.

Les éléments haptiques de l'implant intraoculaire 1' comportent principalement des anses 13' qui s'étendent dans le même sens (par exemple dans le sens des aiguilles d'une montre dans la vue de face de la figure 4) à partir de l'élément optique 10'.

Dans cet exemple, l'implant 1' comporte deux anses 13' diamétralement opposées.

Deux portions annulaires périphériques 15' s'étendent en outre partiellement autour de l'élément optique 10', en vis-à-vis de chaque anse 13' de manière à définir un logement 16' en forme de gorge courbe.

Les portions annulaires périphériques 15' sont en outre raccordées à l'élément optique 10' par un plan incliné 17', sensiblement en regard d'une extrémité libre 13'a de chaque anse 13', c'est-à-dire au niveau de l'entrée du logement 16' tel que défini précédemment.

Des trous de manipulation 14' peuvent en outre être prévus aux extrémités des anses 13' pour permettre d'effectuer la rotation nécessaire de l'implant intraoculaire 1' lors de son montage sur un implant principal 2' comme cela sera expliqué ultérieurement.

L'élément optique 10' de l'implant 1' a un diamètre optique compris entre 5 et 7 mm, et de préférence égal, dans cet exemple, à 5,8 mm.

L'implant 1' lui-même pris dans son ensemble, avec les anses 13', est inscrit dans un cercle de diamètre compris entre 6 et 9 mm.

Dans cet exemple, le diamètre du cercle correspond sensiblement à la distance séparant les extrémités libres 13'a des anses 13' diamétralement opposées.

Comme illustré à la figure 7, les éléments haptiques 13', 14', 15' tels que décrits ci-dessus s'étendent sensiblement dans le plan de l'élément optique 10'.

On va décrire à présent, en référence à la figure 5, un implant principal 2' sur lequel l'implant intraoculaire 1' décrit précédemment peut être fixé.

Cet implant principal 2' est ici, à titre d'exemple non limitatif, un implant formant lentille intraoculaire monofocale 1' adapté à remplacer le cristallin.

De manière classique, cet implant principal 2' comprend un élément optique 20', qui est ici, à titre d'exemple non limitatif, constitué d'une lentille biconvexe de puissance comprise par exemple entre 10 et 30 dioptries.

Sa fonction est de remplacer optiquement le cristallin, lorsque celui-ci est retiré, notamment s'il est atteint de la cataracte.

Le diamètre optique de cet élément optique 20' est compris par exemple entre 5 et 7 mm.

Cet implant principal 2' comporte des éléments haptiques 23' qui permettent de fixer l'implant 2' à l'intérieur de la chambre postérieure de l'oeil comme illustré à la figure 7.

Ces éléments haptiques 23' forment des anses d'appui, régulièrement réparties à la périphérie de d'élément optique 20' et s'étendant chacune sur un secteur angulaire inférieur à 90°.

L'implant principal 2' comporte ici deux anses 23', diamétralement opposées, ayant chacune une première extrémité 21' de raccordement à la périphérie de la partie optique 20', et une seconde extrémité libre 22' adaptée à venir en appui élastique entre la capsule antérieure et la capsule postérieure du sac capsulaire ou éventuellement dans le sulcus ciliaire.

Les anses 23' étant dans un même sens (ici le sens inverse des aiguilles d'une montre dans la vue de face de la figure 5) à partir de l'élément optique 20'.

Bien entendu, d'autres formes haptiques pourraient être utilisées pour monter l'implant principal 2' dans la chambre postérieure de l'oeil.

Comme illustré à la figure 7, les extrémités de raccordement 21' de chaque anse 23' s'étendent dans un plan transversal au plan de l'élément optique 20' passant par un diamètre de cet élément optique 20'.

Les extrémités de raccordement 21' sont en outre angulées antérieurement par rapport au plan optique de l'élément optique 20' et sont antérieurement divergentes.

L'angulation est telle que la distance séparant l'extrémité 22' des anses 23' augmente plus on s'éloigne du plan optique de l'élément optique 20'.

Ces extrémités de raccordement 21' sont prolongés par des anses classiques 23', sensiblement en forme de C, qui s'étendent dans un plan incliné par rapport au plan optique, avec une angulation comprise par exemple entre 5° et 15°.

Le diamètre total D de cette implant principal 2', correspondant ici à la distance séparant les extrémités libres 22' des anses 23' est compris entre 9 et 13 mm, et de préférence égal à 12,5 mm.

L'implant principal 2' et l'implant intraoculaire 1' conforme à l'invention, peuvent être en tout type de matériau pourvu qu'ils procurent une bonne stabilité après l'implantation.

En outre, comme décrit précédemment, l'implant intraoculaire 1' est de préférence réalisé dans un matériau rigide à indice de réfraction élevé, afin de limiter l'inclinaison nécessaire du prisme pour un angle de déviation fixé.

Conformément à ce mode de réalisation, et comme illustré aux figures 6 et 7, les éléments haptiques 13' de l'implant intraoculaire 1' sont adaptés à coopérer avec les éléments haptiques 23' de l'implant principal 2'. Ainsi, comme bien illustré à la figure 6, les anses 13' et 23' des deux implants s'entrecroisent de telle sorte que les anses 23', et plus particulièrement leurs extrémités de raccordement 21' soient logées dans le logement 16' des éléments haptiques 13' de l'implant intraoculaire prismatique 1'.

Lors de l'introduction des anses 23' dans le logement 16, par rotation de l'implant intraoculaire 1', les plans inclinés 17' coopèrent avec les extrémités de raccordement 21' des anses 23' de l'implant principal 2' déjà implanté et jouent un rôle de came pour éloigner l'implant intraoculaire 1' de l'implant principal 2', le long des extrémités de raccordement 21' divergentes des éléments haptiques 23' de l'implant principal 2'.

On obtient ainsi une fixation stable et durable des deux implants 1', 2', sans contact de leurs éléments optiques 10' et 20'.

Afin de limiter encore tout risque de contact des éléments optiques 10' et 20', dans cet exemple de réalisation comme illustré à la figure 7, la portion prismatique de l'élément optique 10' est dirigée antérieurement dans l'oeil.

Le système d'accrochage de l'implant 1' sur l'implant monofocal 2' étant tel que seul un mouvement de rotation d'un implant par rapport à l'autre peut provoquer le décrochage, il n'y a aucun risque de désolidarisation des implants dans la chambre postérieure de l'oeil car seul un mouvement radial des implants peut éventuellement se produire sous l'effet de la rétraction capsulaire.

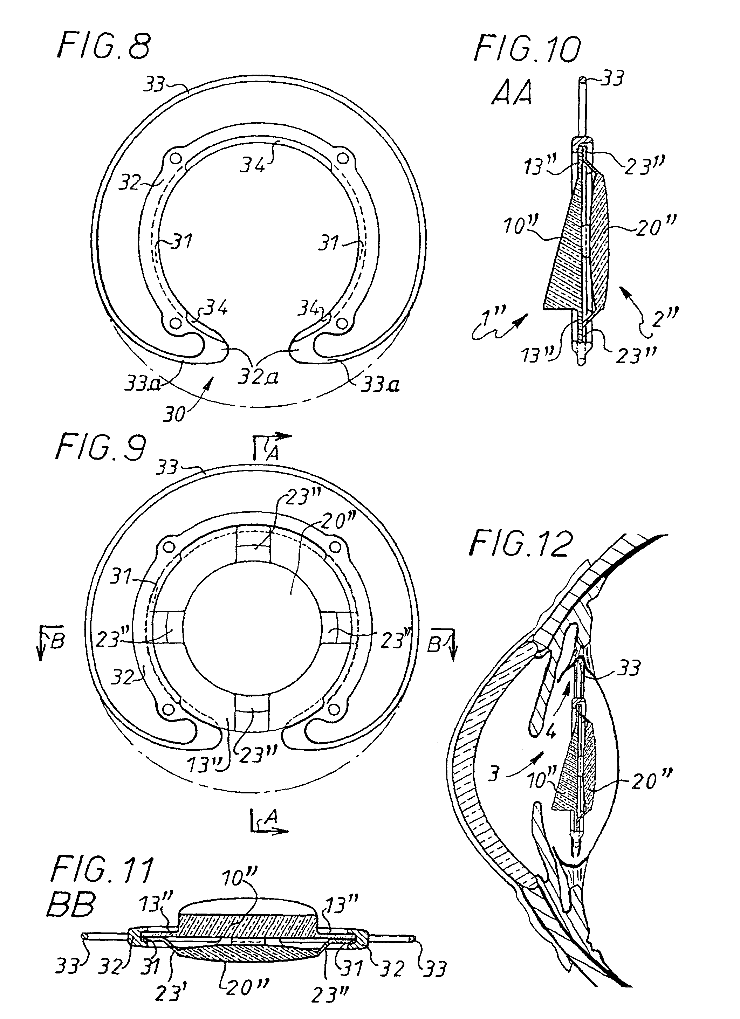

On va décrire à présent un troisième mode de réalisation de l'invention en référence aux figures 8 à 12.

Comme illustré à la figure 8, l'implantation est réalisée en utilisant un support 30 qui permet de monter deux implants côte à côte dans l'oeil.

Ce support 30 comporte une gorge 31 réalisée dans un anneau circulaire 32.

La gorge 31 s'étend ainsi sur la périphérie intérieure de l'anneau 32.

Une branche circulaire concentrique 33 s'étend autour de l'anneau 32.

L'anneau 32 est interrompu sur une portion d'arc de cercle et ses extrémités 32a sont solidaires des extrémités 33a de la branche circulaire 33.

Les dimensions de cette branche circulaire 33 sont telles que le support 30 soit en contact avec les parois de la chambre postérieure de l'oeil lorsqu'il est implanté dans l'oeil.

L'anneau 32 comporte en outre au moins une échancrure 34 qui débouche dans la gorge 31.

Cette échancrure 34 s'étend dans le plan du support et facilite l'introduction des implants dans la gorge 31 du support 30.

Comme illustré aux figures 9 à 11, dans ce mode de réalisation, deux implants 1" et 2" sont montés dans le support 30.

Le premier implant 1" forme une lentille intraoculaire prismatique.

Il comprend comme précédemment un élément optique 10" constitué uniquement d'une portion prismatique et des éléments haptiques 13" qui sont adaptés à s'insérer dans la gorge 31 du support 30.

Ces éléments haptiques 13" s'étendent sensiblement dans le plan de l'élément optique 10".

Un second implant 2" est également adapté à être monté dans le support 30.

Ce second implant 2" est un implant principal formant lentille intraoculaire monofocale adaptée à remplacer fonctionnellement le cristallin.

Ce second implant 2" comporte des éléments haptiques 23" qui sont adaptés à s'insérer dans la gorge 31 du support 30.

Comme bien illustré aux figures 10 et 11, les dimensions de la gorge 31 sont telles que les éléments haptiques 13", 23" des deux implants 1" et 2" peuvent être logés de manière juxtaposée, sensiblement sans jeu, dans la gorge 31 du support 30.

Comme dans le deuxième mode de réalisation, les éléments haptiques 23" du second implant 2" ont une portion inclinée par rapport au plan de l'élément optique 20" de manière à déporter cet élément optique 20" par rapport au plan contenant les extrémités des éléments haptiques 23" destinées à être introduites dans la gorge 31 du support 30.

Ainsi, les éléments optiques 10", 20" des deux implants 1", 2" ne viennent pas en contact l'un avec l'autre.

Les matériaux utilisés pour réaliser ces deux implants 1", 2" sont identiques à ceux utilisés dans les autres modes de réalisation décrits précédemment.

On va décrire à présent le procédé de double implantation des implants principaux 2, 2' et des implants prismatiques 1,1' décrits précédemment, pour l'un ou l'autre des modes de réalisation.

Après extraction du cristallin, on met en place tout d'abord l'implant principal 2, 2' formant ici une lentille monofocale, par chirurgie classique, les dimensions des éléments haptiques 23' leur permettant de venir en appui élastiquement dans le sac capsulaire et éventuellement dans le sulcus ciliaire.

On peut ensuite, soit dans une deuxième opération, soit au cours de la même opération, monter une lentille intraoculaire 1, 1' ayant un fonctionnement prismatique lui permettant de rediriger le faisceau lumineux vers une zone maculaire saine de l'oeil.

Soit cet implant prismatique 1 est monté indépendamment de l'implant monofocal 2, par exemple dans le sulcus ciliaire 5, soit il est juxtaposé à cet implant monofocal 2' par croisement de leurs haptiques respectives 13', 23'.

Ce montage séparé de l'implant principal 2, 2' et de l'implant prismatique conforme à l'invention 1, 1' permet, en cas de dégénérescence maculaire accentuée, de changer l'implant intraoculaire 1, 1' et la correction prismatique appliquée dans l'oeil sans toucher au premier implant 1, 1' corrigeant l'amétropie.

Dans le troisième mode de réalisation, les deux implants 1", 2" sont montés dans le support 30 et celui-ci est implanté comme illustré à la figure 12, dans la chambre postérieure de l'oeil 3, par exemple dans le sac capsulaire 4.

L'implant principal 2" est situé postérieurement dans l'oeil par rapport au premier implant prismatique 1".

Si la dégénérescence maculaire se modifie, on peut changer l'implant prismatique 1" pour modifier la correction prismatique sans toucher au second implant 2" corrigeant l'amétropie.

Bien entendu, de nombreuses modifications peuvent être apportées aux exemples de réalisation décrits ci-dessus, sans sortir du cadre de l'invention.

Ainsi, les élément haptiques 13' de l'implant intraoculaire 1' pourraient avoir une forme différente dès lors qu'ils permettent la fixation de l'implant 1' sur un premier implant monofocal 2'.

## Revendications

1. Implant intraoculaire prismatique pour chambre postérieure de l'oeil, comprenant un élément optique (10, 10', 10") et des éléments haptiques (13, 13', 13") s'étendant à la périphérie de l'élément optique (10, 10', 10"), **caractérisé en ce que** l'élément optique (10, 10', 10") ne comporte que la portion prismatique et est adapté uniquement à dévier un faisceau optique sur une zone maculaire saine.

2. Ensemble d'implants intraoculaires comprenant un implant intraoculaire prismatique selon la revendication 1, **caractérisé en ce qu'**il comporte en outre un implant principal adapté à remplacer le cristallin et à être implanté dans la chambre postérieure (3) de l'oeil.

3. Ensemble d'implants intraoculaires comprenant un implant intraoculaire prismatique selon la revendication 1 ou 2, **caractérisé en ce qu'**il comporte en outre un implant principal (2, 2', 2") adapté à remplacer le cristallin et à être implanté dans le sac capsulaire.

4. Ensemble d'implants intraoculaires comprenant un implant intraoculaire prismatique selon la revendication 1 ou 2, **caractérisé en ce qu'**il comporte en outre un implant principal (2, 2', 2") adapté à remplacer le cristallin et à être implanté dans le sulcus ciliaire.

5. Ensemble d'implants intraoculaires selon l'une des revendications 3 ou 4, comprenant un implant intraoculaire prismatique, **caractérisé en ce que** l'implant principal (2, 2', 2") est un implant formant lentille intraoculaire monofocale (20, 20', 20").

6. Ensemble d'implants intraoculaires selon l'une des revendications 2 à 5, **caractérisé en ce que** les éléments haptiques (13') sont adaptés à coopérer avec des éléments haptiques (23') de l'implant principal (2').

7. Ensemble d'implants intraoculaires selon la revendication 6, **caractérisé en ce que** les éléments haptiques sont des anses (13') de l'implant intraoculaire prismatique adaptées à s'entrecroiser avec des éléments haptiques (23') en forme d'anse de l'implant principal (2').

8. Implant intraoculaire prismatique selon la revendication 1 ou ensemble d'implants intraoculaires selon l'une des revendications 2 à 7, **caractérisé en ce que** l'élément opique (10, 10', 10") de l'implant intraoculaire prismatique est réalisé en un matériau d'indice de réfraction élevé.

## Patentansprüche

1. Prismenförmiges Intraokular-Implantat für die hintere Augenkammer, mit einem optischen Element (10, 10', 10") und haptischen Elementen (13, 13', 13"), die sich längs des Umfangs des optischen Elements (10, 10', 10") erstrecken, **dadurch gekennzeichnet, dass** das optische Element (10, 10', 10") nur den prismenförmigen Abschnitt umfasst und einzig dazu ausgelegt ist, ein Lichtstrahlenbündel auf eine gesunde makuläre Zone abzulenken.

2. Gesamtheit von Intraokular-Implantaten, die ein prismenförmiges Intraokular-Implantat nach Anspruch 1 umfasst, **dadurch gekennzeichnet, dass** sie außerdem ein Hauptimplantat umfasst, das so ausgelegt ist, dass es die Augenlinse ersetzt und dass es in die hintere Augenkammer (3) zu implantieren ist.

3. Gesamtheit von Intraokular-Implantaten, die ein prismenförmiges Intraokular-Implantat nach Anspruch 1 oder 2 umfasst, **dadurch gekennzeichnet, dass** sie außerdem ein Hauptimplantat (2, 2', 2") umfasst, das so ausgelegt ist, dass es die Augenlinse ersetzt und dass es in den Kapselsack zu implantieren ist.

4. Gesamtheit von Intraokular-Implantaten, die ein prismenförmiges Intraokular-Implantat nach Anspruch 1 oder 2 umfasst, **dadurch gekennzeichnet, dass** sie außerdem ein Hauptimplantat (2, 2', 2") umfasst, das so ausgelegt ist, dass es die Augenlinse ersetzt und dass es in den Ziliensulkus zu implantieren ist.

5. Gesamtheit von Intraokular-Implantaten nach einem der Ansprüchen 3 oder 4, die ein prismenförmiges Intraokular-Implantat umfasst, **dadurch gekennzeichnet, dass** das Hauptimplantat (2, 2', 2") ein Implantat ist, das eine monofokale Intraokular-Linse (20, 20', 20") bildet.

6. Gesamtheit von Intraokular-Implantaten nach einem der Ansprüche 2 bis 5, **dadurch gekennzeichnet, dass** die haptischen Elemente (13') so beschaffen sind, dass sie mit den haptischen Elementen (23') des Hauptimplantats (2') zusammenwirken.

7. Gesamtheit von Intraokular-Implantaten nach Anspruch 6, **dadurch gekennzeichnet, dass** die haptischen Elemente Schlingen (13') des prismenförmigen Intraokular-Implantats sind, die mit den schlingenförmigen haptischen Elementen (23') des Hauptimplantats (2') verschlungen sein können.

8. Prismenförmiges Intraokular-Implantat nach Anspruch 1 oder Gesamtheit von Intraokular-Implantaten nach einem der Ansprüche 2 bis 7, **dadurch gekennzeichnet, dass** das optische Element (10, 10', 10") des prismenförmigen Intraokular-Implantats aus einem Werkstoff mit hohem Brechungsindex verwirklicht ist.

## Claims

1. Prismatic intraocular implant for the posterior chamber of the eye, comprising an optical element (10, 10', 10") and haptic elements (13, 13', 13") extending from the periphery of the optic element (10, 10', 10"), **characterised in that** the optic element (10, 10', 10") comprises only the prismatic portion and is adapted solely to divert an optical beam onto a healthy macular area.

2. Set of intraocular implants comprising a prismatic intraocular implant according to Claim 1, **characterised in that** it also comprises a main implant adapted to replace the crystalline lens and to be implanted in the posterior chamber (3) of the eye.

3. Set of intraocular implants comprising a prismatic intraocular implant according to Claim 1 or 2, **characterised in that** it also comprises a main implant (2, 2', 2") adapted to replace the crystalline lens and to be implanted in the capsular sac.

4. Set of intraocular implants comprising a prismatic intraocular implant according to Claim 1 or 2, **characterised in that** it also comprises a main implant (2, 2', 2") adapted to replace the crystalline lens and to be implanted in the ciliary sulcus.

5. Set of intraocular implants according to one of Claims 3 or 4, comprising a prismatic intraocular implant, **characterised in that** the main implant (2, 2', 2") is an implant forming a monofocal intraocular lens (20, 20', 20").

6. Set of intraocular implants according to one of Claims 2 to 5, **characterised in that** the haptic elements (13') are adapted to cooperate with haptic elements (23') of the main implant (2').

7. Set of intraocular implants according to Claim 6, **characterised in that** the haptic elements are loops (13') of the prismatic intraocular implant adapted to intertwine with haptic elements (23') in the form of loops of the main implant (2').

8. Prismatic intraocular implant according to Claim 1 or set of intraocular implants according to one of Claims 2 to 7, **characterised in that** the optical element (10, 10', 10") of the prismatic intraocular implant is produced from a material with a high refractive index.
